# EUROPÄISCHE PATENTSCHRIFT

(11) **EP 2 373 601 B1**
(45) Veröffentlichungstag und Bekanntmachung des Hinweises auf die Patenterteilung: **21.11.2012**
(21) Anmeldenummer: 09768012.8
(22) Anmeldetag: 30.11.2009
(51) Int. Cl.: C07D 313/04, C07C 67/08, C07C 29/149, C07C 31/20, C07C 69/675, C07C 69/44

(54) **VERFAHREN ZUR HERSTELLUNG VON 1,6-HEXANDIOL**
METHOD FOR PRODUCING 1,6-HEXANEDIOL
PROCÉDÉ DE PRÉPARATION DE 1,6-HEXANEDIOL

(30) Priorität: 05.12.2008 EP 08170853
(43) Veröffentlichungstag der Anmeldung: 12.10.2011
(73) Patentinhaber: BASF SE, 67056 Ludwigshafen (DE)
(72) Erfinder: ABILLARD, Olivier, 68161 Mannheim (DE); Dr. TEBBEN, Gerd-Dieter, 68239 Mannheim (DE); PINKOS, Rolf, 67098 Bad Dürkheim (DE); WABNITZ, Tobias, 68169 Mannheim (DE)
(86) Internationale Anmeldenummer: PCT/EP2009/066011
(87) Internationale Veröffentlichungsnummer: WO 2010/063659

(56) Entgegenhaltungen:
- EP-B1- 0 883 591
- WO-A1-2004/046072
- DE-A1- 2 060 548
- DE-A1- 19 607 955
- DE-B- 1 235 879
- US-A- 3 933 930

## Beschreibung

Die Erfindung betrifft ein Verfahren zur Herstellung von 1,6-Hexandiol, bevorzugt mit mindestens 99 %iger Reinheit, die insbesondere von 1,4-Cyclohexandiolen praktisch frei sind, aus einem Carbonsäuregemisch, das als Nebenprodukt der Oxidation von Cyclohexan zu Cyclohexanon/Cyclohexanol mit Sauerstoff oder Sauerstoff enthaltenden Gasen und durch Wasserextraktion des Reaktionsgemisches erhalten wird, durch Veresterung und Hydrierung zu Hexandiol, wobei die Ausbeute an Wertprodukten dadurch gesteigert wird, dass nach einer Veresterungsstufe, bei der Katalysatoren, die mindestens ein Element der Gruppen 3 - 14 enthalten, eingesetzt werden, Glycerin zugesetzt wird.

1,6-Hexandiol stellt einen gesuchten Monomerbaustein dar, der überwiegend auf dem Polyester- und Polyurethansektor eingesetzt wird.

Die wässrigen Lösungen von Carbonsäuren, die bei der Oxidation von Cyclohexan zu Cyclohexanol und Cyclohexanon (vgl. Ullmann's Encyclopedia of Industrial Chemistry, 5. Ed., 1987, Vol. A8, S. 49) als Nebenprodukte entstehen, im folgenden Dicarbonsäurelösung (DCL) genannt, enthalten (berechnet wasserfrei in Gew.-%) im allgemeinen zwischen 10 und 40 % Adipinsäure, zwischen 10 und 40 % 6-Hydroxycapronsäure, zwischen 1 und 10 % Glutarsäure, zwischen 1 und 10 % 5-Hydroxyvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und γ-Butyrolacton genannt.

Aus DE 2 321 101 und DE 1 235 879 ist bekannt, diese wässrigen Dicarbonsäurelösungen bei Temperaturen von 120 bis 300°C und Drücken von 50 bis 700 bar in Gegenwart überwiegend Kobalt enthaltender Katalysatoren zu 1,6-Hexandiol als Hauptprodukt zu hydrieren. Die Hydrierausträge werden bevorzugt destillativ aufgearbeitet. Dabei gelingt es auch mit extrem hohem Destillationsaufwand nicht oder nur unvollständig, die bei der Hydrierung nicht veränderten 1,4-Cyclohexandiole von hexandiol zu trennen, so dass sich die 1,4-Cyclohexandiole, die anfänglich bereits in der DCL enthalten waren, mit einem Gehalt von im allgemeinen 2 bis 5 Gew.-% im 1,6-Hexandiol wiederfinden.

Um diesem Problem zu begegnen, sind einige Lösungsansätze bekannt:

In US 3 933 930 wird die Umsetzung von 1,4-Cyclohexandiol in wässrigen Lösungen von Adipinsäure und 6-Hydroxycapronsäure zu Cyclohexanol, Cyclohexan und/oder Cyclohexen beschrieben, indem das Gemisch katalytisch vorhydriert wird. Dieses Verfahren bedarf des Einsatzes zweier verschiedener Hydrierkatalysatoren, eines für die Vorhydrierung, eines für die eigentliche Carbonsäurehydrierung und ist daher aufwendig.

Nach DE-OS 2 060 548 wird sehr reines 1,6-Hexandiol durch Kristallisation gewonnen. Auch dieses Verfahren ist sehr aufwendig und außerdem mit erheblichen Ausbeuteverlusten verbunden.

Eine weitere Möglichkeit zur Gewinnung von hochreinem 1,6-Hexandiol besteht darin, anstelle von DCL reine Adipinsäure oder reine Adipinsäureester zu hydrieren (K. Weissermel, H.J. Arpe, Industrielle Organische Chemie, VCH-Verlagsgemeinschaft Weinheim, 4. Auflage, Seite 263, 1994). Reine Adipinsäure ist jedoch im Vergleich zu DCL sehr teuer. Ferner ist das Carbonsäuregemisch, das bei der Cyclohexanoxidation anfällt, ein Abfallprodukt, das auch aus Umweltschutzgesichtspunkten einer stofflichen Verwertung zugeführt werden sollte.

In EP-B 883591 ist ein elegantes Verfahren beschrieben, das die Herstellung von 1,6-Hexandiol und Caprolacton in hohen Reinheiten gewährleistet. Nachteilig an diesem Verfahren ist die nicht optimale Ausnutzung der im Carbonsäuregemisch (DCL), das durch Cyclohexanoxidation erhalten wurde, enthaltenen Komponenten Adipinsäure und 6-Hydroxycapronsäure, da diese zum Teil das Verfahren nicht als 1,6-Hexandiol bzw. Caprolacton verlassen, sondern als hochsiedende oligomere Ester.

DE-OS 196 07 955 beschreibt eine Variante des in EP-B 883591 beschriebenen Verfahrens zur Herstellung von 1,6-Hexandiol aus DCL ohne Kopplung mit einer Caprolacton-Gewinnung. WO 2004/046072 betrifft eine weitere Variante dieses Verfahrens, bei dem unter Zugabe eines Acetalisierungsmittels vor dem Hydrierungsschritt sehr reines 1,6-Hexandiol gewonnen wird.

Es bestand daher die Aufgabe sehr reines 1,6-Hexandiol aus der in der DCL enthaltenen Adipinsäure zu gewinnen sowie die Ausbeute an 1,6-Hexandiol zu erhöhen und damit die Nachteile des Standes der Technik, d.h. entweder hohe Kosten der Herstellung oder unzureichende Reinheit der Produkte, zu vermeiden.

Diese Aufgabe wurde gelöst mit einem Verfahren zur Herstellung von 1,6-Hexandiol aus einem Adipinsäure, 6-Hydrocapronsäure und geringe Mengen 1,4-Cyclohexandiole enthaltenden Carbonsäuregemisch, das als Nebenprodukt der Oxidation von Cyclohexan zu Cyclohexanon/Cyclohexanol mit Sauerstoff oder Sauerstoff enthaltenden Gasen und durch Wasserextraktion des Reaktionsgemisches erhalten wird, durch Veresterung und Hydrierung zu Hexandiol, dadurch gekennzeichnet, dass man
a) die in dem wässrigen Reaktionsgemisch enthaltenen Mono- und Dicarbonsäuren mit einem Alkohol mit 1 bis 10 C-Atomen zu den entsprechenden Carbonsäureestem umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eines weniger als 0,5 Gew.-% 1,4-Cyclohexandiole enthaltende Esterfraktion und eine zumindest den größeren Teil der 1,4-Cyclohexandiole enthaltende Fraktion durchführt,
d) die Esterfraktion aus (c) katalytisch hydriert und durch Destillation des Hydrierproduktes in an sich bekannter Weise 1,6-Hexandiol gewinnt,
e) gegebenenfalls das Sumpfprodukt der Stufe c) einer weiteren Veresterungsreaktion mit einem Alkohol mit 1 bis 10 C-Atomen unterzieht, diesen Alkohol nach erfolgter Veresterungsreaktion destillativ entfernt, anschließend aus dem verbleibenden Sumpfstrom Adipinsäurediester und 6-Hydroxycapronsäureester von 1,4-Cyclohexandiolen und Hochsiedern weitgehend destillativ trennt und diese der Stufe c) zuführt.
dadurch gekennzeichnet, dass mindestens eine der Veresterungsreaktionen mit einem Katalysator durchgeführt wird, der mindestens ein Element der Gruppen 3 - 14 enthält, und nach der Veresterungsreaktion Glycerin zugesetzt wird.

Nach dem Schritt c) des erfindungsgemäßen Verfahrens kann in einer dritten Destillationsstufe zumindest teilweise einen im Wesentlichen 6-Hydroxycapronsäuremethylester enthaltenden Strom abgetrennt werden und dieser auf Temperaturen über 200°C bei vermindertem Druck erhitzt werden, wodurch 6-Hydroxycapronsäureester zu Caprolacton cyclisiert wird und aus dem Cyclisierungsprodukt durch Destillation reines ε-Caprolacton gewonnen werden kann.

In Schritt c) des erfindungsgemäßen Verfahren, enthält die weniger als 0,5 Gew.-% 1,4- Cyclohexandiolen enthaltende Esterfraktion bevorzugt weniger als 0,2 Gew.% 1,4-CHDO, besonders bevorzugt weniger als 0.1 Gew.% 1.4-CHDO.

Der Begriff in Schritt c) des erfindungsgemäßen Verfahrens, dass in einer zweiten Destillationsstufe die Abtrennung in eine zumindest den größeren Teil der 1,4-Cyclohexandiole enthaltende Fraktion erfolgt, bedeutet, dass diese Fraktion mehr als 75Gew.-% der sich vor der zweiten Destillationsstufe im Sumpfprodukt befindlichen Anteile an 1,4-Cyclohexandiole enthält.

Es war überraschend, dass bei der Trennung der Estergemische, die durch die Veresterung der in der DCL enthaltenen Mono- und Dicarbonsäuren entstehen, die 1,4-Cyclohexandiole, die ebenfalls mit Carbonsäuren verestert vorliegen können, so abgetrennt werden können, dass nach Hydrierung und Aufarbeitung dem verbleibenden sehr geringen 1,4-Cyclohexandiolgehalt im 1,6-Hexandiol keine praktische Bedeutung mehr zukommt. Wegen der zu trennenden komplizierten Stoffgemische war es überraschend, dass es gelang, die 1,4-Cyclohexandiole oder deren Ester trotz der ungünstigen Siedepunktverhältnisse und zu befürchtenden Azeotropbildung, praktisch vollständig von den für die Hydrierung zu 1,6-Hexandiol verwendeten C₆-Estern abzutrennen. Des Weiteren war es überraschend, dass durch Zugabe von Glycerin nach mindestens einer Veresterungsstufe die Ausbeute an C6-Wertprodukten wie 1,6-Hexandiol und/oder Caprolacton gesteigert werden konnte. Dabei war ebenfalls überraschend, dass keine Zersetzungsprodukte des Glycerins entstanden, die in weiteren Verfahrensschritten zu Problemen führen können, sei es aufgrund von Aufpegelungen oder aufgrund von Komponenten, die toxisch wirken oder weil sie in 1,6-Hexandiol und/oder Caprolacton neue Nebenkomponenten darstellen.

Mindestens eine Veresterung des erfindungsgemäßen Verfahrens wird in Gegenwart eines Katalysators durchgeführt, der ein Übergangsmetall enthält, ansonsten kann die Veresterung ohne Zusatz von Katalysatoren, bevorzugt unter Einwirkung von Katalysatoren durchgeführt werden. Als Alkohole mit 1 bis 10 C-Atomen kommen bevorzugt Alkohole mit 1 bis 8 C-Atomen, besonders bevorzugt Alkohole mit 1 bis 3 C-Atomen in Betracht. Auch Diole wie Butandiol oder Pentandiol kommen prinzipiell in Betracht.

Die technisch bevorzugten für die Veresterung zu verwendenden Alkohole sind Methanol, n- oder i-Butanol und ganz besonders bevorzugt Methanol.

Im Falle der Veresterung mit Methanol geht man so vor, dass man in der Destillationsstufe (c) eine im Wesentlichen von 1,4-Cyclohexandiolen freie Carbonsäuremethylesterfraktion am Kopf der Kolonne und eine die Hochsieder und die 1,4-Cyclohexandiole enthaltende Sumpffraktion gewinnt und die Carbonsäuremethylesterfraktion gemäß Schritt d) weiter verwendet. Des Weiteren besteht die Möglichkeit das erfindungsgemäße Verfahren so zu variieren, dass ein Teil der Carbonsäuremethylesterfraktion auch zur Herstellung von ε-Caprolacton genutzt wird.

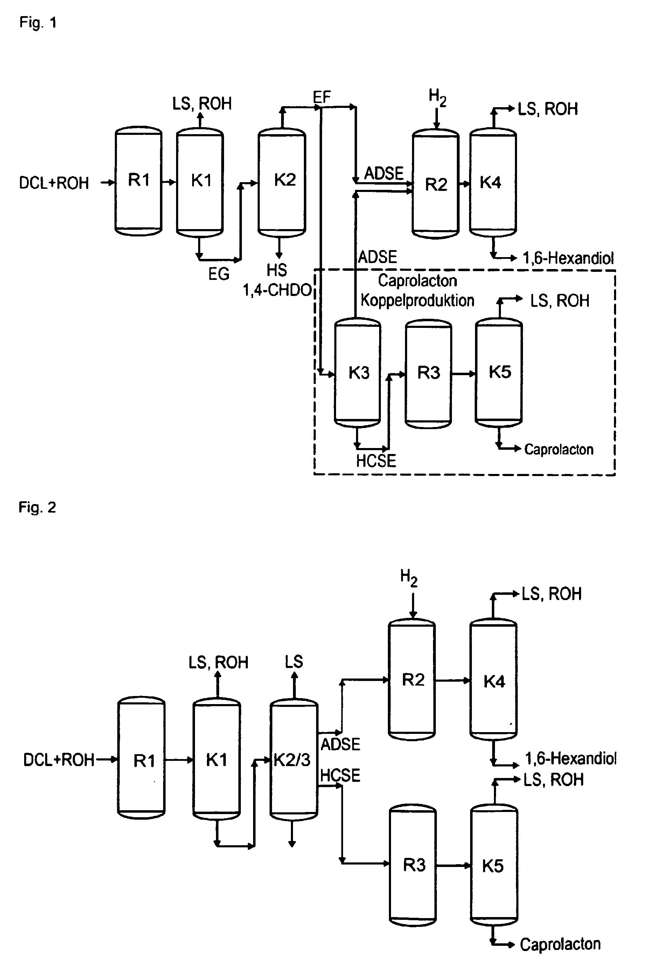

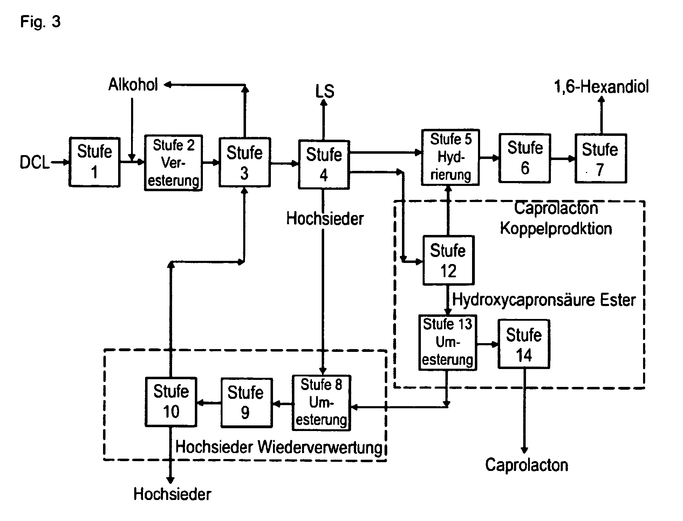

Im Falle der Veresterung mit einem besonders bevorzugten Alkohol mit 1 bis 3 C-Atomen, wird das erfindungsgemäße Verfahren wie folgt allgemein als Variante A erläutert (wobei die Begriffe über Kopf bzw. als Sumpf jeweils den Abzug oberhalb bzw. unterhalb des Zulaufs bedeuten). Figur 1 und 3 geben das erfindungsgemäße Verfahren nach der Varianten A wieder, wobei sowohl die weitere Verwertung der aus Schritt c) entstanden im Wesentlichen 1,4 Cyclohexandiol enthaltenden Fraktion gemäß Schritt e) als auch die dem Schritt c) folgende destillative Abtrennung eines im Wesentlichen 6- Hydroxycapronsäureester enthaltenden Strom und seine weitere Verarbeitung zu Caprolacton mit abgebildet ist. Im Falle einer weiteren Verarbeitung des im Wesentlichen 6-Hydroxycapronsäureester enthaltenden Strom zu Caprolacton, kann das erfindungsgemäße Verfahren nach der Variante B betrieben werden. Figur 2 gibt das erfindungsgemäße Verfahren nach Variante B an.

### Variante A

Wie in Fig. 1 dargestellt, wird die Dicarbonsäurelösung (DCL), gegebenenfalls nach Entwässerung, zusammen mit einem C₁- bis C₃-Alkohol, vorzugsweise Methanol, in den Veresterungsreaktor R₁ eingespeist, in dem die Carbonsäuren verestert werden. Das erhaltene Veresterungsgemisch gelangt dann in die Kolonne K₁, in der der überschüssige Alkohol (ROH), Wasser und Leichtsieder (LS) über Kopf abdestilliert und das Estergemisch (EG) als Sumpf abgezogen und in die Kolonne K₂ eingespeist wird. In dieser Kolonne wird das EG in eine im Wesentlichen von 1,4-Cyclohexandiolen freie Esterfraktion (EF) und eine Sumpffraktion bestehend aus Hochsiedern (HS) und cis-und trans-1,4-Cyclohexandiolen (1,4-CHDO) fraktioniert, die gegebenenfalls gemäß Stufe f) des erfindungsgemäßen Verfahrens weiter aufgearbeitet wird. Die Esterfraktion wird anschließend in der katalytischen Hydrierung R2 zu 1,6-Hexandiol hydriert, das in der Kolonne K4 reindestilliert wird.

Aus dem erfindungsgemäßen Verfahren kann zusätzlich noch £-Caprolacton gewonnen werden. Dafür wird das Verfahren nach Variante A wie folgt modifiziert: Die Esterfraktion aus der Kolonne K2 wird dann in einen weitere Fraktionierkolonne K₃ geleitet, in der die Esterfraktion in ein Kopfprodukt, bestehend im Wesentlichen aus Adipinsäurediester (ADSE), vorzugsweise -dimethylester und ein Sumpfprodukt bestehend im Wesentlichen aus 6-Hydroxycapronsäureester (HCSE), vorzugsweise -methylester aufgetrennt wird.

Die 6-Hydroxycapronsäureesterfraktion aus dem Sumpfprodukt der Fraktionierkolonne K₃ kann im Reaktor R₃ einer thermischen Behandlung über 100°C, in der Regel 150 bis 350°C, vorzugsweise 200 bis 300°C bei vermindertem Druck, z.B. 900 bis 10 mbar, vorzugsweise 300 bis 20 mbar unterworfen werden; dies führt zur Cyclisierung des Esters unter Bildung von ε-Caprolacton, das in der Kolonne K₅ reindestilliert werden kann.

### Variante B:

Gemäß dieser Variante wird die Destillation der Kolonnen K₂ und K₃ zu einer Destillationsstufe zusammengezogen.

Gemäß Fig. 2 wird dabei das durch Veresterung mit Alkoholen mit 1 bis 3 C-Atomen, vorzugsweise Methanol, erhaltene Estergemisch (EG) einer fraktionierenden Destillation unterworfen und in einem oberen Seitenabzug der Adipinsäureester, vorzugsweise -dimethylester, in einem unteren Seitenabzug der 6-Hydroxycapronsäureester, vorzugsweise -methylester, und als Sumpf die 1,4-Cyclohexandiole sowie andere oliogmeren Hochsieder gewonnen. Die Sumpffraktion wird gemäß Stufe f) weiter aufgearbeitet.

Die Adipinsäureester- und 6-Hydroxycapronsäureesterfraktionen werden dann wie in Fig. 1 beschrieben aufgearbeitet.

Das erfindungsgemäße Verfahren wird im Folgenden für die Variante A im Einzelnen anhand der Fig. 3 erläutert. Die Reaktionsbedingungen gelten für die andere Variante gleichermaßen.

Die Verfahrensschritte sind in Figur 3 in Stufen aufgeschlüsselt, wobei die Stufen 2, 3, 4, 5, 6, 7 für das Verfahren essentiell sind, die Stufen 8, 9, 10 zur Steigerung der Ausbeute - sofern notwendig - fakultativ sind und die Stufen 3 und 4 sowie 6 und 7 auch zusammengefasst werden können. Die Stufe 11 ist fakultativ, aber zur Erhöhung der Wirtschaftlichkeit des Verfahrens gegebenenfalls sinnvoll. Falls mit der während des erfindungsgemäßen Verfahrens anfallenden 6-Hydroxycapronsäureesterfraktion auch noch Caprolacton hergestellt werden soll, sind auch die Schritte 12-14 essentiell.

Die Dicarbonsäurelösung (DCL) ist im Allgemeinen eine wässrige Lösung mit einem Wasseranteil von 20 bis 80 %. Da eine Veresterungsreaktion eine Gleichgewichtsreaktion darstellt, bei der Wasser entsteht, ist es sinnvoll, insbesondere bei Veresterung mit z.B. Methanol vorhandenes Wasser vor der Reaktion zu entfernen, vor allem wenn während der Veresterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders 30 bis 200°C und einem Druck von 1 bis 1 500 mbar, bevorzugt 5 bis 1 100 mbar, besonders bevorzugt 20 bis 1 000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, dass das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Die Abtrennung des Wassers kann so erfolgen, dass das Wasser überwiegend säurefrei erhalten wird oder man kann die in der DCL enthaltenen niederen Monocarbonsäuren - im Wesentlichen Ameisensäure - zum größten Teil mit dem Wasser abdestillieren, damit diese in der Veresterung keinen Veresterungsalkohol binden.

Dem Carbonsäurestrom aus der Stufe 1 wird Alkohol ROH mit 1 bis 10 C-Atomen zugemischt. Dabei können Methanol, Ethanol, Propanol oder iso-Propanol oder Gemische der Alkohole, bevorzugt aber Methanol verwendet werden. Das Mischungsverhältnis Alkohol zu Carbonsäurestrom (Massenverhältnis) kann von 0,1 bis 50, bevorzugt 0,2 bis 40, besonders bevorzugt 0,5 bis 30 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400°C, bevorzugt 70 bis 300°C, besonders bevorzugt 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Ebenfalls möglich ist eine Reaktionskolonne, bei der im Gegenstrom Dicarbonsäurelösung und Alkohol so miteinander umgesetzt werden, dass die DCL im oberen Teil der Reaktionskolonne eingebracht wird und nach unten sinkt, Alkohol meist in gasförmigen Zustand von untern eingebracht wird und nach oben steigt. Über Kopf wird überschüssiger Alkohol zusammen mit Reaktionswasser abgezogen, über Sumpf die veresterten Carbonsäuren. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen, bevorzugt wird aber zur Erhöhung der Reaktionsgeschwindigkeit ein Katalysator zugesetzt. Dabei kann es sich um einen homogenen gelösten oder um einen festen Katalysator handeln. Als homogene Katalysatoren seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z.B. Aluminium-, Vanadium-, Titan-, Bor-Verbindungen genannt. Bevorzugt sind Mineralsäuren, insbesondere Schwefelsäure. Wird eine Reaktionskolonne eingesetzt, ist eine bevorzugte Variante, Katalysatoren einzusetzen, die Elemente wie z.B. Ti, Zr, V, Hf , Al, Sn, enthalten. Dabei ist weiterhin bevorzugt, einen Teil der Reaktion autokatalytisch den Rest katalytisch zu gewährleisten. Das Gewichtsverhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O3, SnO₂, ZrO₂, Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt abzüglich der gegebenenfalls zugesetzten Säure als Katalysator 0,01 bis 50, bevorzugt 0,1 bis 10. Dabei müssen nicht alle im System vorhandenen Carboxylgruppen als Ester des eingesetzten Alkohols vorliegen, sondern ein Teil kann in Form von dimeren oder oligomeren Estern z.B. mit dem OH-Ende der Hydroxycapronsäure vorliegen.

Das Veresterungsgemisch wird in Stufe 3, bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet.

Wenn bei der Veresterung des erfindungsgemäßen Verfahrens mittels eines Katalysators, der mindestens ein Element der Gruppen 3 - 14 enthält, die Säurezahl unter 1 mg KOH/g liegt, bevorzugt unter 0,2 ganz bevorzugt unter 0,1, so wird vor dem Eintritt des Produktstroms in eine Kolonne der Stufe 3, spätestens aber der Stufe 4 Glycerin zuzugeben. Das Glycerin wird in Mengen von 0,01 bis 20 Gew.-%, bevorzugt 0,05 bis 5 Gew.-%, besonders bevorzugt 0,1 bis 3 Gew.-% bezogen auf den zu destillierenden Strom eingesetzt. Es kann auch in Form von Gemischen eingesetzt werden, in reiner Form oder auch in gelöster Form. Geeignete Lösemittel sind z.B. Wasser, der Veresterungsalkohol, bevorzugt Methanol, des Weiteren Glykole oder Glycolether oder Tetrahydrofuran.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1 500 mbar, bevorzugt 20 bis 1 000 mbar, besonders bevorzugt 40 bis 800 mbar und Temperaturen zwischen 0 und 150°C, bevorzugt 15 und 90_{°}C und insbesondere 25 und 75°C der überschüssige Veresterungsalkohole ROH, Wasser sowie entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren wie Adipinsäure und Glutarsäure, Hydroxycarbonsäuren wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 4 Gew.% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf zwischen den leichtsiedenden Komponenten und den schwersiedenden Komponenten erfolgt. Die Kolonne wird bei Temperaturen von 10 bis 300 °C, bevorzugt 20 bis 270 °C, besonders bevorzugt 30 bis 250 °C und Drucken von 1 bis 1000 mbar, bevorzugt 5 bis 500 mbar, besonders bevorzugt 10 bis 200 mbar betrieben.

Die Kopffraktion besteht überwiegend aus Restwasser und Restalkohol ROH, Estern des Alkohols ROH mit Monocarbonsäuren, überwiegend C₃- bis C₆-Monocarbonsäureestern mit Hydroxycarbonsäuren, wie 6-Hydroxycapronsäure, 5-Hydroxyvaleriansäure sowie vor allem den Diestern mit Dicarbonsäuren wie Adipinsäure, Glutarsäure und Bernsteinsäure, 1,2-Cyclohexandiolen, Caprolacton und Valerolacton. Diese von 1,4-Cyclohexandiolen im Wesentlichen freie Kopffraktion enthält weniger als 0,5 Gew.% 1,4-CHDO, bevorzugt weniger als 0,2 Gew.% 1,4-CHDO, besonders bevorzugt weniger als 0,1 Gew.% 1,4-CHDO.

Die genannten Komponenten können zusammen über Kopf abgetrennt oder in einer weiteren bevorzugten Ausführungsform in der Kolonne der Stufe 4 in einen Kopfstrom, der überwiegend Restwasser und Restalkohol sowie die oben erwähnten Bestandteile mit 3 bis 5 C-Atomen enthält und einen Seitenstrom, der überwiegend die oben erwähnten Bestandteile der C₆-Ester enthält, aufgetrennt werden. Der die Ester der C₆-Säuren enthaltende Strom, entweder als Gesamt-Kopfstrom oder als Seitenstrom kann dann, je nachdem wie viel Caprolacton hergestellt werden soll, im erfindungsgemäßen Verfahren - ohne Caprolacton-Herstellung - ganz in die Hydrierung (Stufe 5) gelangen, wahlweise jedoch zum Teil oder als Gesamtstrom in die Stufe 12 eingespeist werden.

Die schwersiedenden Komponenten des Stromes aus Stufe 4, überwiegend bestehend aus 1,4-Cyclohexandiolen oder deren Estern, dimeren oder oligomeren Estern sowie nicht näher definierte z.T. polymeren Bestandteilen der DCL, werden über den Abtriebsteil der Kolonne der Stufe 4 abgetrennt. Diese können zusammen anfallen oder so, dass über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Sofern die Ausbeute an gewünschten C6-Estern nach dem efindungsgemäßen Verfahren bereits hoch genug ist, können die Stufen 8, 9 und 10 entfallen. Sollten aber die beschriebenen Vorstufen nicht erfindungsgemäß ausgestaltet werden, d.h. die Veresterung nicht mit einem Katalysator, der mindestens ein Element der Gruppen 3 - 14 enthält, gefolgt von der Zugabe von Glycerin durchgeführt werden, dann sind die Stufen 8 bis 10 notwendig.

Dazu wird in dem erfindungsgemäßen Verfahren das Sumpfprodukt der Stufe 4 einer weiteren Veresterungsreaktion unterzogen. Da in dieser Stufe 8 ganz überwiegend oligomere Ester in monomere Ester mittels eines Alkohols und eines Katalysators überführt werden, wird diese Stufe auch "Umesterungsstufe" bezeichnet.

Dazu wird in der Stufe 8 der Anteil an dimeren und oligomeren Estern der Adipinsäure bzw. Hydroxycapronsäure mit weiteren Mengen des Alkohols ROH, vorzugsweise Methanol, in Gegenwart Katalysators, der mindestens ein Element der Gruppen 3 - 14 enthält, umgesetzt. Das Gewichtsverhältnis von Alkohol ROH und dem Sumpfstrom aus Stufe 4 beträgt zwischen 0,1 bis 20, bevorzugt 0,5 bis 10, besonders bevorzugt 1 bis 5. Als Katalysatoren eignen sich Verbindungen oder Komplexe beispielsweise des Aluminiums, Zinns, Antimons, Zirkons oder Titans, wie Zirkoniumacetylacetonat oder Tetraalkyltitanat wie Tetraisopropyltitanat, die in Konzentrationen von 1 bis 10 000 ppm, bevorzugt 50 bis 6 000 ppm, besonders bevorzugt 100 bis 4 000 ppm, angewandt werden. Besonders bevorzugt sind Titanverbindungen.

Die Umesterung kann absatzweise oder kontinuierlich, in einem Reaktor oder mehreren Reaktoren, in Reihe geschaltenen Rührkesseln oder Rohrreaktoren oder einer Reaktionskolonne bei Temperaturen zwischen 100 und 300 °C, bevorzugt 120 bis 270 °C, besonders bevorzugt 140 bis 240 °C und den sich dabei einstellenden Eigendrücken, durchgeführt werden. Die benötigten Verweilzeiten liegen bei 0,5 bis 10 Stunden, bevorzugt bei 1 bis 4 Stunden.

Der Reaktionsaustrag der Stufe 8 wird in einer nachfolgenden Destillation von überschüssigem ROH befreit (Stufe 9), wobei im Falle von ROH = Methanol das Methanol über Kopf abgetrennt wird. Erfindungsgemäß wird vor dem Eintritt des Produktstroms in eine Kolonne der Stufe 9, spätestens aber der Stufe 10 Glycerin zugeben. Das Glycerin wird in Mengen von 0,01 bis 20 Gew.-%, bevorzugt 0,05 bis 5 Gew.-%, besonders bevorzugt 0,1 bis 3 Gew.-% bezogen auf den zu destillierenden Strom eingesetzt. Es kann auch in Form von Gemischen eingesetzt werden, in reiner Form oder auch in gelöster Form. Geeignete Lösemittel sind z.B. Wasser, der Veresterungsalkohol, bevorzugt Methanol, des Weiteren Glykole oder Glykolether oder Tetrahydrofuran.

In der Stufe 9, wird die Kolonne so betrieben, dass der Zulauf zur Kolonne bevorzugt zwischen dem Kopf- und dem Sumpfstrom erfolgt.

Über Kopf wird bei Drücken von 1 bis 1 500 mbar, bevorzugt 20 bis 1 000 mbar, besonders bevorzugt 40 bis 800 mbar und Temperaturen zwischen 0 und 150°C, bevorzugt 15 und 90°C und insbesondere 25 und 75°C der überschüssige Veresterungsalkohol abgezogen und beispielsweise in die Stufe 11 oder in die Stufe 2 oder Stufe 8 rückgeführt.

Der Sumpfstrom der Stufe 9 wird in die Stufe 10, ebenfalls eine Destillationskolonne, überführt. Dabei handelt es sich um eine Destillationskolonne, bei der der Zulauf zwischen den leichtsiedenden Komponenten und den schwersiedenden Komponenten erfolgt. Die Kolonne wird bei Temperaturen von 10 bis 300 °C, bevorzugt 20 bis 270°C, besonders bevorzugt 30 bis 250 °C und Drucken von 1 bis 1000 mbar, bevorzugt 5 bis 500 mbar, besonders bevorzugt 10 bis 200 mbar betrieben.

Über Kopf wird im Wesentlichen ein Gemisch aus Adipinsäurediester und 6-Hydroxycapronsäureester gewonnen, im Sumpf sind überwiegend 1,4-Cyclohexandiole und zum Großteil unbekannte Hochsieder, sowie das zugesetzte Glycerin enthalten. Das Kopfprodukt der Stufe 10 kann entweder der Stufe 4 oder der Stufe 12 zugeführt werden.

Mit dem erfindungsgemäßen Vorgehen lassen sich die in der DCL vorhandenen Adipinsäure- und 6-Hydroxycapronsäureenheiten in höheren Ausbeuten gewinnen und damit die Ausbeuten an 1,6-Hexandiol und Caprolacton steigern. Das erfindungsgemäße Verfahren wirkt sich insbesondere ausbeutesteigernd auf den 6-Hydroxycapronsäureester aus. So kann die Ausbeute der monomeren C6-Ester beispielsweise um 5 bis 25 % gesteigert werden.

Die Stufen 3 und 4 können, insbesondere wenn nur kleinere Mengen verarbeitet werden, zusammengefasst werden. Dazu kann beispielsweise in einer absatzweise durchgeführten fraktionierten Destillation der C₆-Esterstrom gewonnen werden, wiederum ohne dass 1,4--Cyclohexandiole in den zur Hydrierung geführten Strom gelangen.

Sofern nach dem Verfahren nur 1,6-Hexandiol gewonnen werden soll, kann das Kopfprodukt oder das aus dem Seitenabzug der Stufe 4 ggf. zusammen mit dem Kopfprodukt der Stufe 10 direkt, ohne weitere Aufreinigung in einer Hydrierung umgesetzt werden.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind in H. Kropf, Houben-Weyl, Methoden der Organischen Chemie, Band IV/1c, Georg Thieme Verlag Stuttgart, 1980, S. 45 bis 67, und Beispiele für heterogene Katalysatoren sind in Houben-Weyl, Methoden der Organischen Chemie, Band IV/1c, S. 16 bis 26, beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. Cr₂O₃, Al₂O₃, SiO₂, ZrO₂, TiO₂, ZnO₂, BaO und MgO oder Mischungen daraus.

Bevorzugt werden Heterogenkatalysatoren verwendet, die entweder fest angeordnet oder als Suspension eingesetzt werden. Wird die Hydrierung in der Gasphase und über fest angeordnetem Katalysator durchgeführt, werden im Allgemeinen Temperaturen von 150 bis 300°C bei Drücken von 1 bis 50 bar angewandt. Dabei wird mindestens so viel Wasserstoff als Hydriermittel und Trägergas verwendet, dass Edukte, Zwischenprodukte und Produkte während der Reaktion nie flüssig werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinander geschalteten Reaktoren durchgeführt werden. Die Hydrierung in Flüssigphase über einem Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann diskontinuierlich, bevorzugt kontinuierlich erfolgen.

Der Hydrieraustrag besteht im Wesentlichen aus 1,6-Hexandiol und dem Alkohol ROH. Weitere Bestandteile sind vor allem, falls der gesamte leichtsiedende Strom der Stufe 4 eingesetzt wurde, 1,5-Pentandiol, 1,4-Butandiol, 1,2-Cyclohexandiole sowie kleine Mengen von Monoalkoholen mit 1 bis 6 C-Atomen und Wasser.

Der Hydrieraustrag wird in der Stufe 6, z.B. ein Membransystem oder bevorzugt eine Destillationskolonne, in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1 500 mbar, bevorzugt 30 bis 1 200 mbar, besonders bevorzugt 50 bis 1 000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 2 zurückgeführt werden oder in die Stufe 8 oder in die Stufe 11 gelangen.

Der 1,6-Hexandiol enthaltene Stoffstrom wird in der Stufe 7 in einer Kolonne gereinigt. Dabei werden 1,5-Pentandiol, die 1,2-Cyclohexandiole sowie weitere eventuell vorhandene Leichtsieder über Kopf abgetrennt. Sollen die 1,2-Cyclohexandiole und/oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99 % aus einem Seitenstrom der Kolonne entnommen. Dabei werden bei Drücken von 1 bis 1 000 bar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen 6 und 7 auch in einer diskontinuierlichen fraktionierten Destillation zusammengefasst werden.

Um das erfindungsgemäße Verfahren möglichst wirtschaftlich zu betreiben, ist es sinnvoll, den Veresterungsalkohol ROH zurückzugewinnen und immer wieder zur Veresterung einzusetzen. Dazu kann der vorwiegend den Alkohol ROH enthaltende Strom aus Stufe 3 und/oder 6 in der Stufe 11 aufgearbeitet werden. Dazu wird vorteilhaft eine Kolonne verwendet, in der Komponenten, die leichter sieden als der Alkohol ROH über Kopf, Wasser und Komponenten, die höher sieden als der Alkohol ROH, über Sumpf vom Alkohol ROH, der in einem Seitenstrom gewonnen wird, abgetrennt werden. Die Kolonne wird zweckmäßig bei 500 bis 5 000 mbar, bevorzugt bei 800 bis 3 000 mbar betrieben.

Eine bevorzugte Verfahrensvariante sieht neben der Herstellung von 1,6-Hexandiol auch die Gewinnung von Caprolacton vor. Dazu wird für die Caprolactonherstellung der vorwiegend Ester der C₆-Säuren enthaltende Strom aus der Stufe 4 eingesetzt. Dazu wird dieser Strom in Stufe 12, einer Destillationskolonne, in einen überwiegend Adipinsäurediester enthaltenden Strom, der die vorhandenen 1,2-Cyclohexandiole enthält, über Kopf und einen überwiegend 6-Hydroxycapronsäureester enthaltenden Strom, der keine 1,2-Cyclohexandiole enthält, über Sumpf aufgetrennt. Die Kolonne wird bei Drücken von 1 bis 500 mbar, bevorzugt 5 bis 350 mbar, besonders bevorzugt 10 bis 200 mbar und Sumpftemperaturen von 80 bis 250°C, bevorzugt 100 bis 200°C, besonders bevorzugt 110 bis 180°C betrieben. Die Kopftemperaturen stellen sich dabei entsprechend ein.

Wichtig für eine hohe Reinheit und hohe Ausbeute an Caprolacton ist die Abtrennung der 1,2-Cyclohexandiole vom Hydroxycapronsäureester, da diese Komponenten Azeotrope miteinander bilden. Es war in dieser Stufe 12 nicht vorauszusehen, dass die Trennung der 1,2-Cyclohexandiole und des Hydroxycapronsäureesters vollständig gelingt, vor allem wenn als Ester der bevorzugte Methylester eingesetzt wird.

Der 6-Hydroxycapronsäureester enthaltende Sumpfstrom der Stufe 12 wird in der Stufe 13 in an sich bekannter Weise entweder in der Gas- oder Flüssigphase zu Alkohol und Caprolacton umgesetzt. Bevorzugt ist die Flüssigphase.

Die Reaktion wird ohne Katalysator oder aber bevorzugt in Anwesenheit eines Katalysators durchgeführt. Als Katalysatoren eignen sich saure oder basische Katalysatoren, die homogen gelöst oder heterogen vorliegen können. Beispiele sind Alkali- und Erdalkalimetallhydroxide-, -oxide, -carbonate, -alkoxylate oder -carboxylate, Säuren wie Schwefel- oder Phosphorsäure, organische Säuren wie Sulfonsäuren oder Mono- oder Dicarbonsäuren, bzw. Salze der vorgenannten Säuren, Lewissäuren, bevorzugt aus der III. und IV. Hauptgruppe bzw. der I. bis VIII. Nebengruppe des Periodensystems der Elemente.

Bevorzugt verwendet man die gleichen Katalysatoren, die auch in der Stufe 8 eingesetzt werden, da der hochsiedende Ausschleusstrom der Stufe 13 oligomere Hydroxycapronsäureeinheiten enthält, die vorteilhaft über die Stufe 8 wiederverwertet werden können. Wird ein heterogener Katalysator eingesetzt, so beträgt die Katalysatorbelastung üblicherweise 0,05 bis 5 kg Edukt/I Katalysator und Stunde. Bei homogenen Katalysatoren wird der Katalysator bevorzugt dem Eduktstrom zugemischt. Dabei beträgt die Konzentration üblicherweise 10 bis 10000 ppm, bevorzugt 50 bis 5000 ppm, besonders bevorzugt 100 bis 1000 ppm. Die Reaktion wird üblicherweise bei 150 bis 400°C, bevorzugt 180 bis 350°C, besonders bevorzugt 190 bis 330°C und Drücken von 1 bis 1020 mbar, bevorzugt 5 bis 500 mbar, besonders bevorzugt 10 bis 200 mbar durchgeführt.

In manchen Fällen ist es vorteilhaft die Cyclisierungsreaktion in Gegenwart von hochsiedenden Mono-, Di- oder Polyolen wie z.B. Decanol, Undecanol, 1,4-Butandiol, 1,5-Pentandiol, 1,6-Hexandiol, 1,4-Cyclohexandiolen oder Glycerin durchführen.

Diese hochsiedenden Alkohole oder Polyole werden vorgelegt oder dem Reaktionsgemisch zugesetzt, jeweils in Konzentrationen von 1 und 20000 ppm, bevorzugt 10 bis 4000 ppm, besonders bevorzugt 50 bis 2000 ppm.

Wird die Cyclisierung in der Flüssigphase durchgeführt, werden die Reaktionsprodukte, vorwiegend Veresterungsalkohol ROH und Caprolacton gasförmig aus dem Reaktionsgemisch entfernt. Vorteilhaft ist eine dem Reaktionsgefäß aufgesetzte Kolonne, in der noch nicht umgesetztes Edukt im Reaktionssystem gehalten werden kann und über Kopf der Alkohol und Caprolacton abgezogen werden. Dabei kann die Kondensation des Produktstroms so erfolgen, dass fraktioniert kondensiert wird, d.h. zuerst vorwiegend Caprolacton, dann der Veresterungsalkohol. Selbstverständlich kann auch nur der Alkohol über Kopf, Caprolacton dagegen in einem Seitenstrom gewonnen werden. Der Alkohol-Strom kann vorteilhaft in die Stufe 2, 8 oder 11 zurückgeführt werden. Das Sumpfprodukt der Cyclierung kann in die Stufe 8 eingeschleust werden.

Der Caprolacton-Produktstrom der Stufe 13 wird in der Stufe 14 weiter aufgearbeitet. Dabei kann es sich um eine oder mehrere Kolonnen handeln. Wird eine Kolonne verwendet, so werden über Kopf der gegebenenfalls noch vorhandene Veresterungsalkohol, sowie andere C₁- bis C₆-Leichtsieder abgetrennt, über Seitenstrom reines Caprolacton und über den Sumpf gegebenenfalls noch nicht umgesetzter Hydroxycapronsäureester, der zurückgeführt wird.

Hochreines Caprolacton erhält man, wenn in der Stufe 14 die erwähnten Leichtsieder in einer ersten Kolonne über Kopf, Caprolacton und andere Hochsieder über Sumpf in eine zweite Kolonne eingespeist werden, wo Caprolacton über Kopf abgezogen wird. Handelt es sich bei dem zu gewinnenden Caprolactonstrom nur um kleinere Mengen, so kann Caprolacton mit einer Kolonne durch absatzweise fraktionierte Destillation gewonnen werden.

Die Destillationen werden bei Sumpftemperaturen von 70 bis 250°C, bevorzugt 90 bis 230°C, besonders bevorzugt 100 bis 210°C und Drücken von 1 bis 500 mbar, bevorzugt 5 bis 200 mbar, besonders bevorzugt 10 bis 150 mbar durchgeführt.

Nach dem erfindungsgemäßen Verfahren werden Ausbeuten an 1,6-Hexandiol und Caprolacton von jeweils über 95 %, bei Reinheiten von über 99 % erzielt.

Das Verfahren wird anhand der nachfolgenden Beispiele näher erläutert. Den Beispielen der Serie 1 liegt die Veresterung in einem Strömungsrohr mit Schwefelsäure als Katalysator zugrunde und die Serie unterteilt sich in erfindungsgemäß und nicht erfindungsgemäß. Den Beispielen der Serie 2 ist eine Veresterung in einer Reaktionskolonne zugrunde gelegt.

### Beispiele Serie 1

### Beispiel a (Vergleichsbeispiel):

### Stufe 1: (Entwässerung)

0,1 kg/h Dicarbonsäurelösung (Adipinsäure, 6-Hydroxycapronsäure, 1,4-Cyclohexandiole, Glutarsäure, 5-Hydroxyvaleriansäure, Ameisensäure, Wasser) wurden kontinuierlich in einer Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (insgesamt 6 kg) betrug der Wassergehalt ca. 0,4 %.

### Stufe 2: (Veresterung)

5,5 kg des Sumpfstroms aus Stufe 1 wurde mit 8,3 kg Methanol und 14 g Schwefelsäure ca. 120°C in einem Strömungsrohr bei einer Verweilzeit von ca. 3 Stunden umgesetzt. Die Säurezahl des Austrags abzüglich Schwefelsäure betrug ca. 10 mg KOH/g.

### Stufe 3:

In einer Kolonne wurde der Veresterungsstrom aus Stufe 2, aus dem die Schwefelsäure abgetrennt worden war, destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 4: (1,4-Cyclohexandiolabtrennung)

In einer 50 cm Füllkörperkolonne wurde der Sumpfstrom aus Stufe 3 fraktioniert destilliert (1 mbar, 70-90°C Kopftemperatur, bis 180°C Sumpftemperatur). Im Sumpf fanden sich neben unbekannten Hochsiedern dimere und oligomere Ester auf Basis Adipinsäure und 6-Hydroxycapronsäure sowie die 1,4-Cyclohexandiole.

Als Leichsieder wurden 0,6 kg abdestilliert (1,2-Cyclohexandiole. Valerolacton, 5-Hydroxyvaleriansäuremethylester, Glutarsäuredimethylester, Bemsteinsäuredimethylester u.a.); als überwiegend Adipinsäuredimethylester und 6-Hydroxycapronsäuremethylester enthaltende Fraktion wurden 4,3 kg erhalten.

### Stufe 5: (Hydrierung Teilstrom)

2,7 kg C₆-Estergemisch aus Stufe 4 wurden kontinuierlich in einem 25 ml Reaktor an einem Katalysator hydriert (Katalysator, 70 Gew.-%, CuO, 25 Gew.-% ZnO, 5 Gew.-% Al₂O₃. der zuvor im Wasserstoffstrom bei 180°C aktiviert worden ist, Hydrierbedingungen: Zulauf 20 g/h, keim Umlauf, 220 bar, 220°C). Der Ester-Umsatz betrug 99,5 %, die 1,6-Hexandiolselektivität betrug über 99 %.

### Stufe 6 und 7: (Hexandiolreinigung)

2,5 kg des Hydrieraustrags aus Stufe 5 wurden fraktioniert destilliert (Destillationsblase mit aufgesetzter 70 cm Füllkörperkolonne, Rücklaufverhältnis 2). Bei 1013 mbar wurden 0,5 kg Methanol abdestilliert und nach Anlegen von Vakuum (20 mbar) destillierten überwiegend die 1,2-Cyclohexandiole und 1,5-Pentandiol ab. Danach (Sdp. 146°C) destillierte 1,6-Hexandiol mit einer Reinheit von 99,8 % ab.

### Stufe 8:

2,9 kg des Sumpfaustrages der Stufe 4 wurden mit 3,8 kg Methanol und 3,8 g Tetra-i-propyltitanat versetzt und kontinuierlich in einem 1 m langen, 440 ml fassenden Rohrreaktor, der mit 3 mm V2A-Ringen gefüllt war, umgesetzt. Die mittlere Verweilzeit betrug ca. 2 h.

### Stufe 9:

Der Austrag aus Stufe 8 wurde analog der in Stufe 3 beschriebenen Apparatur fraktioniert destilliert. Bei 65°C Kopftemperatur wurden 3,5 kg abdestilliert (überwiegend Methanol). Im Sumpf verblieben 2,2 kg.

### Stufe 10:

Der Sumpfstrom aus der Stufe 9 wurde in einer Kolonne kontinuierlich destilliert. Der Zulauf (80 g/h) wurde oberhalb des Sumpfes zugeführt. Am Kopf wurde das gewonnene Destillat anteilig rückgeführt (Rücklaufverhältnis 1,3:1). Der Sumpfstand wurde mittels Ventil und kontinuierlicher Sumpfausschleusung konstant gehalten. Die Destillationsapparatur wurde unter folgenden Bedingungen betrieben: Druck 18 mbar, Sumpftemperatur 180 °C, Kopftemperatur 110 °C.
Das gewonnene Destillat (35 g/h) bestand hauptsächlich aus 6-Hydroxycapronsäuremethylester (48 Gew.%) und Adipinsäuredimethylester (29 Gew.%). Die Hochsieder - u.a. bestanden überwiegend aus unbekannten Komponenten, 1,4-Cyclohexandiole und oligomere Ester auf Basis 6-Hydroxycapronsäure und Adipinsäure. Die Ausbeute an 6-Hydroxycapronsäuremethylester der Stufe 10 betrug 65% bzw. 99 % an Adipinsäuredimethylester bezogen auf den Anteil an monomeren Ester im Sumpf der Stufe 9.

### Stufe 11:

7 kg des Kopfproduktes der Stufe 3 wurden an einer 20 cm Füllkörperkolonne bei 1015 mbar fraktioniert destilliert. Es wurden 0,8 kg Vorlauffraktion bei 59 - 65°C Kopftemperatur erhalten, die neben vorwiegend Methanol, C₁- C₄-Monoethylester enthielt. Bei 65°C Kopftemperatur wurden 5,6 kg Methanol mit einer Reinheit > 99 % erhalten. Der Sumpf (0,6 kg) bestand überwiegend aus Wasser.

### Stufe 12:

Von 2,0 kg Estergemisch aus Stufe 4 und dem Kopfprodukt der Stufe 10 wurden in einer 4 I Destillationsblase mit aufgesetzter Kolonne (40 cm, 5 mm V2A-Metallringfüllkörper) und Rücklaufteiler bei 2 mbar vorwiegend Adipinsäuredimethylester und 6-Hydroxycapronsäuremethylester abdestilliert (Rücklaufverhältnis 2, Kopftemperatur bis 91°C, Sumpftemperatur bis 118°C). Im Sumpf blieben 0,5 kg Hydroxycapronsäuremethylester (85 %ig, Rest vorwiegend dimerer Hydroxycapronsäuremethylester, kein Adipinsäuredimethylester) zurück.

### Stufe 13: (Cyclisierung)

In einer 250 ml Destillationsblase mit außenliegender Heizung und aufgesetzter Kolonne (70 cm, 5 mm V2A-Metallringfüllkörper) mit Rücklaufteiler wurden 60 ml Sumpfprodukt aus Stufe 12 mit Zusatz von 1000 ppm Tetraisopropyltitanat vorgelegt, auf 260°C bei 40 mbar aufgeheizt und stündlich 35 ml Sumpfprodukt aus Stufe 12, dem 1000 ppm Tetraisopropyltitanat sowie 200 ppm 1,6-Hexandiol zugesetzt wurden, zugefahren. Bei einer Kopftemperatur von 123 bis 124°C und einem Rücklaufverhältnis von 4 wurden bei 25°C vorwiegend Caprolacton, bei -78°C Methanol kondensiert.

### Stufe 14: (Caprolactonreinigung)

In einer 250 ml Destillationsblase mit aufgesetzter Kolonne (70 cm, 5 mm V2A-Metallringfüllkörper) und Rücklaufteiler (Rücklaufverhältnis 4) wurde das aus Stufe 13 erhaltene Caprolacton fraktioniert bei 40 mbar destilliert. Nach Abtrennung von im wesentlichen Valerolacton (Sdp. 90 bis 110°C) wurde Caprolacton (Sdp. 131°C) in einer Reinheit (GC-Flächen-%) von 99,9 % erhalten.

### Beispiele Serie 1

### Beispiel b (Erfindungsgemäßes Beispiel):

Das Vorgehen der Stufen 1 bis 14 aus dem Vergleichsbeispiel der Serie 1 wurde wiederholt mit dem Unterschied, dass vor der Stufe 10 1 Gew.-% Glycerin zugegeben wurden. Bei 18 mbar Druck und einer Sumpftemperatur von 180°C stellte sich eine Kopftemperatur von 115°C ein. Das gewonnene Destillat bestand hauptsächlich aus 6-Hydroxycapronsäuremethylester (57 Gew.%) und Adipinsäuredimethylester (25 Gew.%). Die Ausbeute an 6-Hydroxycapronsäuremethylester betrug 90%, die des Adipinsäuredimethylesters 99%.

Das Kopfprodukt wurde analog dem Vergleichsbeispiel Stufe 12, 13 und 14 weiterverarbeitet. Es kam zu keiner Änderung in der Ausbeute bzw. Zusammensetzung des Caprolactons.

### Beispiele Serie 2

### Beispiel c (Vergleichsbeispiel)

### Stufe 1: (Entwässerung)

0,1 kg/h Dicarbonsäurelösung (Adipinsäure, 6-Hydroxycapronsäure, 1,4-Cyclohexandiole, Glutarsäure, 5-Hydroxyvaleriansäure, Ameisensäure, Wasser) wurden kontinuierlich in einer Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (insgesamt 6 kg) betrug der Wassergehalt ca. 0,4 %.

### Stufe 2: (Veresterung)

Insgesamt 6 kg des Sumpfstroms aus Stufe 1 wurde in einer 10-stufigen Glockenbodenkolonne mit 10 verschiedenen äußeren Heizkreisen bei Temperaturen zwischen 180 und 160°C im Gegenstrom kontinuierlich mit Methanol verestert. Dabei wurde das Säuregemisch auf den zweitobersten Glockenboden zugepumpt, Methanol wurde auf den untersten Glockenboden gepumpt und zuvor auf 180°C erhitzt. Auf den 5 Glockenboden wurden bezogen auf den Säurezulauf 2000 ppm Tetra-i-propyltitanat 10%ig in Methanol gelöst zugepumpt. Über Kopf wurde bei einem Rücklaufverhältnis von 5 überwiegend Methanol, Wasser und Leichtsieder wie Methylformiat abgetrennt, über Sumpf wurde ein Estergemisch erhalten, das eine Säurezahl von ca. 0,2 mg KOH/g aufwies und neben den Estern noch ca. 5 % Methanol aufwies.

### Stufe 3:

In einer Kolonne wurden 5 kg des Esterprodukts aus Stufe 2 destilliert (1015 mbar, 65-70°C Kopftemperatur, bis 125°C Sumpftemperatur). Über Kopf wurden ca. 0,5 kg abgezogen, überwiegend Methanol. Als Sumpfprodukt wurden ca. 4,5 kg erhalten.

### Stufe 4: (1,4-Cyclohexandiolabtrennung)

Der Sumpfstrom aus der Stufe 3 wurde in einer Kolonne kontinuierlich destilliert. Der Zulauf wurde oberhalb des Sumpfes zugeführt. Am Kopf wurde das gewonnene Destillat anteilig rückgeführt (Rücklaufverhältnis ca. 1). Der Sumpfstand wurde mittels Ventil und kontinuierlicher Sumpfausschleusung konstant gehalten. Die Destillationsapparatur wurde unter folgenden Bedingungen betrieben: Druck 20 mbar, Sumpftemperatur bis 170 °C, Kopftemperatur bis 105 °C. Im Kopfprodukt fanden sich 1,2-Cyclohexandiole, Valerolacton, 5-Hydroxyvaleriansäuremethylester, Glütarsäuredimethylester, Bernsteinsäuredimethylester, Caprolacton und Adipinsäuredimethylester (ca. 50 % Ausbeute bzgl. des im Zulauf enthaltenen Adipinsäuredimethylesters) sowie 6-Hydroxycapronsäuremethylester (ca. 25 % bezogen auf den im Zulauf enthaltenen 6-Hydroxycapronsäuremethylesters). Es wurden ca. 1,5 kg Destillat erhalten. Das Sumpfprodukt bestand aus den 1,4-Cyclohexandiolen, unbekannten Hochsiedern und dimeren bzw. oligomeren Estern aus Adipinsäure und 6-Hydroxycapronsäure. Insgesamt wurden ca. 2 kg Sumpfprodukt erhalten. Der Rest setzte sich aus hold up in der Kolonne und nicht kondensiertem Methanol zusammen.

Das Kopfprodukt wurde analog der Stufen 5, 6 und 7 hydriert und aufgearbeitet. Es wurde 1,6-Hexandiol in einer Reinheit bis zu 99,8 % erhalten in einer Ausbeute bei dieser Reinheit bezogen auf die nach Stufe 2 vorhandenen 6-Hydroxycapronsäuremethylester und Adipinsäuredimethylester von ca. 20 %.

### Beispiele Serie 2

### Beispiel c (Erfindungsgemäßes Beispiel)

### Stufe 1: (Entwässerung)

0,1 kg/h Dicarbonsäurelösung (Adipinsäure, 6-Hydroxycapronsäure, 1,4-Cyclohexandiole, Glutarsäure, 5-Hydroxyvaleriansäure, Ameisensäure, Wasser) wurden kontinuierlich in einer Destillationsapparatur (dreibödige Glockenbodenkolonne mit außenliegendem Öl--Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,045 kg/h erhalten mit einem Ameisensäuregehalt im Wasser von ca. 3 %. Im Sumpfstrom (insgesamt 6 kg) betrug der Wassergehalt ca. 0,4 %.

### Stufe 2: (Veresterung)

Insgesamt 6 kg des Sumpfstroms aus Stufe 1 wurde in einer 10-stufigen Glockenbodenkolonne mit 10 verschiedenen äußeren Heizkreisen bei Temperaturen zwischen 180 und 160°C im Gegenstrom kontinuierlich mit Methanol verestert. Dabei wurde das Säuregemisch auf den zweitobersten Glockenboden zugepumpt, Methanol wurde auf den untersten Glockenboden gepumpt und zuvor auf 180°C erhitzt. Auf den 5 Glockenboden wurden bezogen auf den Säurezulauf 2000 ppm Tetra-i-propyltitanat 10%ig in Methanol gelöst zugepumpt. Über Kopf wurde bei einem Rücklaufverhältnis von 5 überwiegend Methanol, Wasser und Leichtsieder wie Methylformiat abgetrennt, über Sumpf wurde ein Estergemisch erhalten, das eine Säurezahl von ca. 0,2 mg KOH/g aufwies und neben den Estern noch ca. 5 % Methanol aufwies.

### Stufe 3:

Zum Austrag der Stufe 2 wurde 2 Gew.-% Glycerin gegeben und anschließend wurden 5,1 kg in einer Kolonne destilliert (1015 mbar, 65-69°C Kopftemperatur, bis 125°C Sumpftemperatur). Über Kopf wurden ca. 0,3 kg abgezogen, überwiegend Methanol. Als Sumpfprodukt wurden ca. 4,9 kg erhalten.

### Stufe 4: (1,4-Cyclohexandiolabtrennung)

Der Sumpfstrom aus der Stufe 3 wurde in einer Kolonne kontinuierlich destilliert. Der Zulauf wurde oberhalb des Sumpfes zugeführt. Am Kopf wurde das gewonnene Destillat anteilig rückgeführt (Rücklaufverhältnis ca. 1). Der Sumpfstand wurde mittels Ventil und kontinuierlicher Sumpfausschleusung konstant gehalten. Die Destillationsapparatur wurde unter folgenden Bedingungen betrieben: Druck 20 mbar, Sumpftemperatur bis 170 °C, Kopftemperatur bis 115 °C. Im Kopfprodukt fanden sich 1,2-Cyclohexandiole, Valerolacton, 5-Hydroxyvaleriansäuremethylester, Glutarsäuredimethylester, Bemsteinsäuredimethylester, Caprolacton und Adipinsäuredimethylester (ca. 90 % Ausbeute bzgl. des im Zulauf enthaltenen Adipinsäuredimethylesters) sowie 6-Hydroxycapronsäuremethylester (ca. 80 % bezogen auf den im Zulauf enthaltenen 6-Hydroxycapronsäuremethylesters). Es wurden ca. 3,0 kg Destillat erhalten. Das Sumpfprodukt bestand aus den 1,4-Cyclohexandiolen, unbekannten Hochsiedern und dimeren bzw. oligomeren Estern aus Adipinsäure und 6-Hydroxycapronsäure. Insgesamt wurden ca. 0,9 kg Sumpfprodukt erhalten. Der Rest setzte sich aus hold up in der Kolonne und nicht kondensiertem Methanol zusammen.

Das Kopfprodukt wurde analog der Stufen 5, 6 und 7 hydriert und aufgearbeitet. Es wurde 1,6-Hexandiol in einer Reinheit bis zu 99,8 % erhalten in einer Ausbeute bei dieser Reinheit bezogen auf die nach Stufe 2 vorhandenen 6-Hydroxycapronsäuremethylester und Adipinsäuredimethylester von ca. 80 %.

## Patentansprüche

1. Verfahren zur Herstellung von 1,6-Hexandiol aus einem Adipinsäure, 6-Hydroxycapronsäure und geringe Mengen 1,4-Cyclohexandiole enthaltenden Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhalten wird, durch Veresterung und Hydrierung zu Hexandiol, **dadurch gekennzeichnet, dass** man
a) die in dem wässrigen Reaktionsgemisch enthaltenen Mono- und Dicarbonsäuren mit einem Alkohol mit 1 bis 10 C-Atomen zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine weniger als 0,5 Gew.-% 1,4-Cyclohexandiole enthaltende Esterfraktion und eine zumindest den größeren Teil der 1,4-Cyclohexandiole enthaltende Fraktion durchführt,
d) die Esterfraktion aus (c) aus der zumindest teilweise 6-Hydroxycapronsäureester entfernt wurde, katalytisch hydriert und durch Destillation des Hydrierproduktes in an sich bekannter Weise 1,6-Hexandiol gewinnt,
e) gegebenenfalls das Sumpfprodukt der Stufe c) einer weiteren Veresterungsreaktion mit einem Alkohol mit 1 bis 10 C-Atomen unterzieht, diesen Alkohol nach erfolgter Veresterungsreaktion destillativ entfernt, anschließend aus dem verbleibenden Sumpfstrom Adipinsäurediester und 6-Hydroxycapronsäureester von 1,4-Cyclohexandiolen und Hochsiedern weitgehend destillativ trennt und diese der Stufe c) zuführt,
**dadurch gekennzeichnet, dass** mindestens eine der Veresterungsreaktionen mit einem Katalysator durchgeführt wird, der mindestens ein Element der Gruppen 3 - 14 enthält, und nach der Veresterungsreaktion Glycerin zugesetzt wird.

2. Verfahren gemäß Anspruch 1, **dadurch gekennzeichnet, dass** man das Carbonsäuregemisch vor der Veresterung entwässert.

3. Verfahren gemäß Anspruch 1, **dadurch gekennzeichnet, dass** man die Veresterung mit Alkoholen mit 1 bis 3 Kohlenstoffatomen durchführt.

4. Verfahren gemäß Anspruch 1, **dadurch gekennzeichnet, dass** man die Veresterung mit Alkoholen mit 4 bis 10 Kohlenstoffatomen durchführt.

5. Verfahren gemäß Anspruch 1, **dadurch gekennzeichnet, dass** man die Veresterung mit Methanol durchführt und in der Destillationsstufe (c) eine weniger als 0,5 Gew.-% 1,4-Cyclohexandiole enthaltende Carbonsäuremethylesterfraktion am Kopf der Kolonne und eine die Hochsieder und die 1,4-Cyclohexandiole enthaltende Sumpffraktion gewinnt.

6. Verfahren gemäß Anspruch 1, **dadurch gekennzeichnet, dass** man die Veresterung mit n- oder i-Butanol durchführt und in der Destillationsstufe (c) die 1,4-Cyclohexandiole mit den Leichtsiedern über Kopf abtrennt und die Carbonsäurebutylester als Seitenstrom oder als diese enthaltenden Sumpf gewinnt.

7. Verfahren gemäß Anspruch 5, **dadurch gekennzeichnet, dass** man im Falle der Veresterung mit Methanol in einem oberen Seitenabzug eine im wesentlichen Dicarbonsäuremethylester enthaltende Fraktion, als unteren Seitenabzug eine im wesentlichen 6-Hydroxycapronsäuremethylesterfraktion und als Sumpfprodukt eine die 1,4-Cyclohexandiole enthaltende Fraktion abtrennt.

8. Verfahren gemäß Anspruch 6, **dadurch gekennzeichnet, dass** man im Falle der Veresterung mit n- oder i-Butanol in einem oberen Seitenabzug eine im wesentlichen 6-Hydroxycapronsäurebutylester enthaltende Fraktion, als unteren Seitenabzug eine im wesentlichen Dicarbonsäurebutylester enthaltende Fraktion und als Kopfprodukt eine die 1,4-Cyclohexandiole enthaltende Fraktion gewinnt.

9. Verfahren gemäß einem der Ansprüche 1 bis 8, **dadurch gekennzeichnet, dass** als Katalysator für die Veresterungsreaktion von Schritt e) eine Titanverbindung in einer Konzentration von 1 bis 10.000 ppm verwendet wird.

10. Verfahren gemäß einem der Ansprüche 1 bis 9, **dadurch gekennzeichnet, dass** als Katalysator für die Veresterungsreaktion von Schritt a) eine Titanverbindung verwendet wird.

## Claims

1. A process for preparing 1,6-hexanediol from a carboxylic acid mixture which comprises adipic acid, 6-hydroxycaproic acid and small amounts of 1,4-cyclohexanediols and is obtained as a byproduct of the oxidation of cyclohexane to cyclohexanone/cyclohexanol with oxygen or oxygen-comprising gases by water extraction of the reaction mixture, by esterification and hydrogenation to hexanediol, which comprises
a) reacting the mono- and dicarboxylic acids present in the aqueous reaction mixture with an alcohol having 1 to 10 carbon atoms to give the corresponding carboxylic esters,
b) freeing the resulting esterification mixture of excess alcohol and low boilers in a first distillation stage,
c) in a second distillation stage, performing a separation of the bottom product into an ester fraction comprising less than 0.5% by weight of 1,4-cyclohexanediols and a fraction comprising at least the majority of the 1,4-cyclohexanediols,
d) catalytically hydrogenating the ester fraction from (c), from which 6-hydroxycaproic ester has been at least partly removed, and obtaining 1,6-hexanediol in a manner known per se by distilling the hydrogenation product,
e) optionally subjecting the bottom product of stage c) to a further esterification reaction with an alcohol having 1 to 10 carbon atoms, removing this alcohol by distillation on completion of the esterification reaction, then substantially separating adipic diesters and 6-hydroxycaproic esters from 1,4-cyclohexanediols and high boilers in the remaining bottom stream by distillation and feeding said adipic diesters and 6-hydroxycaproic esters to stage c),
which comprises performing at least one of the esterification reactions with a catalyst which comprises at least one element of groups 3-14, and adding glycerol after the esterification reaction.

2. The process according to claim 1, wherein the carboxylic acid mixture is dewatered before the esterification.

3. The process according to claim 1, wherein the esterification is performed with alcohols having 1 to 3 carbon atoms.

4. The process according to claim 1, wherein the esterification is performed with alcohols having 4 to 10 carbon atoms.

5. The process according to claim 1, wherein the esterification is performed with methanol and, in distillation stage (c), a methyl carboxylate fraction comprising less than 0.5% by weight of 1,4-cyclohexanediols is obtained at the top of the column and a bottom fraction is obtained comprising the high boilers and the 1,4-cyclohexanediols.

6. The process according to claim 1, wherein the esterification is performed with n- or i-butanol and, in distillation stage (c), the 1,4-cyclohexanediols are removed via the top with the low boilers and the butyl carboxylates are obtained as a sidestream or as bottoms comprising them.

7. The process according to claim 5, wherein, in the case of esterification with methanol, a fraction comprising essentially methyl dicarboxylates is removed in an upper side draw, an essentially methyl 6-hydroxycaproate fraction as a lower side draw and a fraction comprising the 1,4-cyclohexanediols as a bottom product.

8. The process according to claim 6, wherein, in the case of esterification with n- or i-butanol, a fraction comprising essentially butyl 6-hydroxycaproate is obtained in an upper side draw, a fraction comprising essentially butyl dicarboxylates as a lower side draw, and a fraction comprising the 1,4-cyclohexanediols as a top product.

9. The process according to any of claims 1 to 8, wherein the catalyst used for at least one of the esterification reactions is a titanium compound.

## Revendications

1. Procédé pour la production de 1,6-hexanediol par estérification et hydrogénation en hexanediol à partir d'un mélange d'acides carboxyliques contenant de l'acide adipique, de l'acide 6-hydroxycaproïque et de faibles quantités de 1,4-cyclohexanediols, qui est obtenu par extraction à l'eau du mélange rédactionnel, en tant que sous-produit de l'oxydation de cyclohexane en cyclohexanone/cyclohexanol avec de l'oxygène ou des gaz contenant de l'oxygène, **caractérisé en ce que**
a) on fait réagir les acides mono- et dicarboxyliques, contenues dans le mélange rédactionnel aqueux, avec un alcool ayant de 1 à 10 atomes de carbone, pour obtenir les esters d'acides carboxyliques correspondants,
b) dans une première étape de distillation on sépare le mélange d'estérification obtenu d'avec l'alcool en excès et les composés à bas point d'ébullition,
c) dans une deuxième étape de distillation on effectue à partir du produit de pied un fractionnement en une fraction ester contenant moins de 0,5 % en poids de 1,4-cyclohexanediols et une fraction contenant au moins la majeure partie des 1,4-cyclohexanediols,
d) on soumet à une hydrogénation catalytique la fraction ester provenant de (c), de laquelle l'ester d'acide 6-hydroxycaproïque a été au moins partiellement éliminé, et on obtient de façon connue du 1,6-hexanediol par distillation du produis d'hydrogénation,
e) éventuellement on soumet le produis de pied de l'étape c) à une nouvelle réaction d'estérification par un alcool avant de 1 à 10 atomes de carbone, une fois effectuée la réaction d'estérification on élimine cet alcool par distillation, ensuite on sépare par distillation du courant de pied prestant, dans une large mesure, le diester d'acide adipique et l'ester d'acide 6-hydroxycaproïque d'avec les 1,4-cyclohexanediols et les composés à haut point d'ébullition et on envoie ces derniers à l'étape c),
**caractérise en ce qu'**on effectue au moins l'une des réactions d'estérification avec un catalyseur qui contient au moins un élément des groupes 3-14, et après la réaction d'estérification on ajoute du glycérol.

2. Procédé selon la revendication 1, **caractérisé en ce qu'**avant l'estérification on déshydrate le mélange d'acides carboxyliques.

3. Procédé selon la revendication 1, **caractérisé en ce qu'**on effectue l'estérification avec des alcools ayant de 1 à 3 atomes de carbone.

4. Procédé selon la revendication 1, **caractérisé en ce qu'**on effectue l'estérification avec des alcools ayant de 4 à 10 atomes de carbone.

5. Procédé selon la revendication 1, **caractérisé en ce qu'**on effectue l'estérification avec du méthanol et dans l'étape de distillation (c) on obtient à la tête de la colonne une fraction ester méthylique d'acide carboxylique contenant moins de 0,5 % en poids de 1,4-cyclohexanediols et une fraction de pied contenant les composés à haut point d'ébullition et les 1,4-cyclohexanediols.

6. Procédé selon la revendication 1, **caractérisé en ce qu'**on effectue l'estérification avec du n-butanol ou de l'isobutanol et dans l'étape de distillation (c) on sépare par la tête les 1,4-cyclohexanediols avec les composés à bas point d'ébullition et on obtient les esters butyliques diacides carboxyliques en tant que courant latéral ou en tuant que courant de pied les contentant.

7. Procédé selon la revendication 5, **caractérisé en ce que** dans le cas de l'estérification par le méthanol on sépare dans un courant de dérivation latéral supérieur une fraction contenant essentiellement des esters méthyliques d'acides dicarboxyliques, en tant que courant de dérivation latéral inférieur une fraction contenant essentiellement de l'ester méthylique d'acide 6-hydroxycaproïque et en tant que produit de pied une fraction contenant les 1,4-cyclohexanediols.

8. Procédé selon la revendication 6, **caractérisé en ce que** dans le cas de l'estérification par le n-butanol ou l'isobutanol on sépare dans un courant de dérivation latéral supérieur une fraction contenant essentiellement l'ester butylique d'acide 6-hydroxycaproïque, en tant que courant de dérivation latéral intérieur une fraction contenant essentiellement des esters butyliques d'acides dicarboxyliques et en tant que produis de tête une fraction contenant les 1,4-cyclohexanediols.

9. Procédé selon l'une quelconque des revendications 1 à 8, **caractérisé en ce qu'**on utilise comme catalyseur pour la réaction d'estérification de l'étape e) un composé de titane à une concentration de 1 à 10 000 ppm.

10. Procédé selon l'une quelconque des revendications 1 à 9, **caractérisé en ce qu'**on utilise comme catalyseur pour la réaction d'estérification de l'étape a) un composé de titane.
